(19) Europäisches Patentamt
European Patent Office
Office européen des brevets

(11) Numéro de publication: **0 228 920**
**B1**

(12) **FASCICULE DE BREVET EUROPEEN**

(45) Date de publication du fascicule du brevet: **03.05.89**

(51) Int. Cl.⁴: **C07D 235/06,** A61K 31/38

(21) Numéro de dépôt: **86402180.3**

(22) Date de dépôt: **03.10.86**

(54) **Nouveau dérivé du thiochromanne, son procédé de préparation et les compositions pharmaceutiques qui le contiennent.**

(30) Priorité: **04.10.85 FR 8514755**

(43) Date de publication de la demande:
**15.07.87 Bulletin 87/29**

(45) Mention de la délivrance du brevet:
**03.05.89 Bulletin 89/18**

(84) Etats contractants désignés:
**AT BE CH DE FR GB IT LI LU NL SE**

(56) Documents cités:
**FR-A- 2 498 930**
**FR-M- 4 161**
**GB-A- 1 561 153**
**GB-A- 2 124 082**

(73) Titulaire: **ADIR ET COMPAGNIE, 22, rue Garnier, F-92200 Neuilly-sur-Seine(FR)**

(72) Inventeur: **Marchand, Bernard, 71 Rue Laveau, F-45430 Checy(FR)**
Inventeur: **Gargouil, Yves Michel, 38 Rue Michel-Ange, F-75016 Paris(FR)**

**EP 0 228 920 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau dérivé du thiochromanne, son procédé de préparation et les compositions pharmaceutiques les renfermant.

On connait certains dérivés du alkylamino-1 (thiochromannyl-8 oxy)-3 propanol possédant d'intéressantes propriétés pharmacologiques, notamment le tert-butylamino-1 (thiochromannyl-8 oxy)-3 propanol-2 décrit dans le brevet français No. 2 092 004 et dans le brevet britannique N° 1 561 153. Le tert-butylamino-1 (thiochromannyl-8 oxy)-3 propanol-2 ou (tert-butylamino-3 hydroxy-2 propyloxy)-8 thiochromanne ou tertatolol appartient à la classe des agents bêta-bloquants et possède des propriétés cardiovasculaires utilisées en particulier dans le traitement de l'hypertension artérielle. La demanderesse a maintenant découvert un nouveau bêta-bloquant dérivé de tert-butylamino-1 (thiochromannyl-8 oxy)-3 propanol possédant une structure chimique très proche du tertatolol et possédant une activité bêta$_1$-adrénolytique deux fois supérieure à celle du tertatolol.

La présente invention a plus particulièrement pour objet un dérivé du thiochromanne, le tert-butylamino-1 (hydroxy-4 thiochromannyl-8 oxy)-3 propanol-2, qui a la formule chimique suivante:

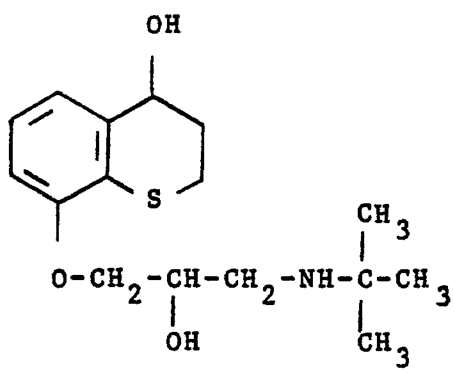

(I)

Ce dérivé présente deux atomes de carbone asymétrique, et il existe donc sous forme racémique et de diastéréoisomères qui font à ce titre partie de l'invention.

L'invention se rapporte également aux sels d'addition du composé de formula I, à un acide minéral ou organique pharmaceutiquement acceptable. Parmi les acides utilisés pour la formation de ces sels, on peut citer les acides phosphorique, chlorhydrique, sulfurique, acétique, propionique, citrique, oxalique, benzoïque etc.

La présente invention a également pour objet le procédé de préparation du composé de formule générale I, caractérisé en ce que l'on réduit le méthoxy-8 thiochromannone-4 de formule II:

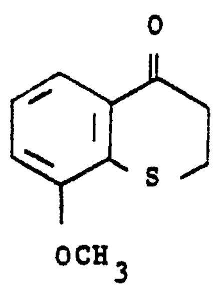

(II)

par un hydrure de bore en méthoxy-8 thiochromannol-4 de formule III:

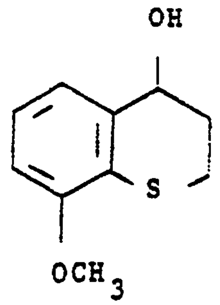

(III)

qu'on déméthyle ensuite, à l'aide d'un thiolate pour obtenir l'hydroxy-8 thiochromannol-4 de formule IV:

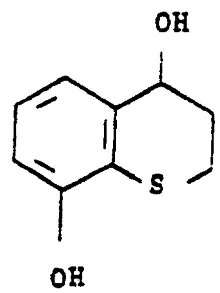

(IV)

sur lequel on fait réagir le chloro-1 époxy-2,3 propane pour former l'(hydroxy-4 thiochromannyl-8 oxy)-3 époxy-1,2 propane de formule V:

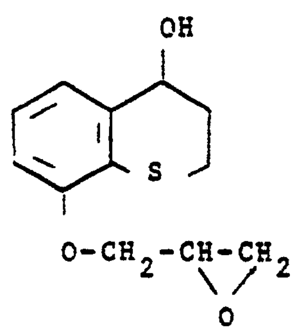

V

et que l'on condense avec la tert-butylamine pour obtenir le composé de formule générale I, et qu'ensuite il est possible de former ses sels d'addition par action d'un acide minéral ou organique pharmaceutiquement acceptable, ou de le séparer à ses diastéréoisomères qui à leur tour peuvent être salifiés.

La méthoxy-8 thiochromannone-4 peut-être préparée en utilisant le procédé décrit par F. Kollpfeiffer et Col., (Ber. (1925), 58, 1654–1676). La réduction de la thiochromannone en thiochromannol et la déméthylation de l'éther-oxyde phénolique sont effectuées par des méthodes déjà connues dans la littérature et décrites respectivement dans Vogel's Textbook of Practical organic chemistry Editions Longman (1978) London, New York, 4edition, p.353–356 et Tetrahedron (1982), 38, 2721–2724 (L.Testaferri et al.). La réaction de l'hydroxy-8 thiochromannol-4 avec le chloro-1 époxy-2,3 propane et la condensation du produit issu de cette réaction avec une amine primaire sont décrites dans le brevet français N° 2 092 004.

Le composé de formule I étant très proche du tertatolol, ses propriétés bêta-bloquantes étaient prévisibles. Par contre, il était inattendu que ce composé possède une activité bêta$_1$-adrénolytique deux fois supérieure à celle du tertatolol. En effet, les essais pharmacologiques in vitro ont montré que le tert-butylamino-1 (hydroxy-4 thiochromannyl-8 oxy)-3 propanol-2 est doué d'une activité bêta$_1$ adrénolytique très importante.

Les propriétés bêta-bloquantes du composé de l'invention permettent donc son application dans le traitement de l'hypertension, de l'angor, de l'ischemie myocardique, des troubles du rythme cardiaque et des manifestations cardio-vasculaires des hyperthyroïdies.

L'invention s'étend aussi aux compositions pharmaceutiques renfermant comme principe actif le composé de formule I, l'un de ses diastéréoisomères ou l'un de ses sels d'addition avec un acide minéral ou organique pharmaceutiquement compatible en association avec un excipient inerte, non toxique et approprié.

Les compositions pharmaceutiques ainsi obtenues sont présentées avantageusement sous des formes diverses telles que par exemple, comprimés, dragées, gélules, glossettes ou autres préparations galéniques appropriées pour une administration sublinguale, suppositoires, solutions injectables ou buvables.

Les compositions pharmaceutiques selon l'invention peuvent en outre contenir un autre principe actif d'action complémentaire ou synergique.

Parmi ces derniers principes actifs, on pourra citer un diurétique et notamment, un saliurétique ou des substances antagonistes calciques.

La posologie peut varier largement en fonction de l'âge, du poids du patient, de la nature et de la sévérité de l'affection ainsi que de la voie d'administration. La voie d'administration préférée est la voie buccale ou parentérale. D'une manière générale, la posologie unitaire s'échelonnera entre 0,1 et 5 mg et la posologie journalière, utilisable en thérapeutique humaine, entre 0,1 et 5 mg.

Les exemples suivants, donnés à titre non limitatif, illustrent l'invention. Les points de fusion sont déterminés au bloc-Köfler.

Exemple 1

Méthoxy-8 thiochromannol-4

On dissout 89 mmol de méthoxy-8 thiochromannone-4 dans 70 ml d'un mélange de méthanol et de tétrahydrofuranne (3/4). On ajoute 134 mmoles de borohydrure de sodium en solution dans 50 ml de méthanol aqueux (50%). Après 1 heure d'agitation à température ambiante puis extraction au dichlorométhane, le produit obtenu est recristallisé dans un mélange de méthanol et d'éther éthylique (1/10).

Rendement 87%.

Point de fusion du produit obtenu 91–93°C.

Exemple 2

Hydroxy-8 thiochromannol-4

On chauffe à 120°C une solution de 25 mmol de méthoxy-8 thiochromannol-4 obtenu précédemment dans 50 ml d'hexaméthylphosphorotriamide (HMPT). On ajoute à chaud 2,5 équivalents d'isopropanethiolate de sodium et on agite pendant 1 heure 30 à 120°C sous azote.

Le mélange refroidi est versé dans 150 ml d'acide chlorhydrique N et extrait par du dichlorométhane. Après lavage de la phase organique jusqu'à disparition de l'HMPT et évaporation du solvant, on recristallise dans un mélange de dichlorométhane et d'éther éthylique (1/1).

On obtient 4,2 g d'hydroxy-8 thiochromannol-4. Rendement 85%.

Exemple 3

Tert-butylamino-1 (hydroxy-4 thiochromannyl-8 oxy)-3 propanol-2

On dissout 48,2 g d'hydroxy-8 thiochromannol-4 dans 150 ml d'acétone. On ajoute 104 ml de chloro-1 époxy-2,3 propane puis 109 g de carbonate de potassium. On porte à reflux pendant 8 heures. On filtre et on évapore jusqu'à poids constant à 50°C sous pression réduite (12mm Hg). On obtient 58 g de mélange d'époxyde et d'halohydrine. On engage 48 g de ce mélange avec 180 ml de tert-butylamine dans 100 ml d'isopropanol.

On porte à reflux 7 heures. Après évaporation de l'amine et du solvent, on reprend le résidu huileux par du dichlorométhane et on lave à l'hydroxyde d'ammonium N.

Après élimination du solvant à 40°C sous pression réduite, on obtient du tert-butylamino-1 (hydroxy-4 thiochromannyl-8 oxy)-3 propanol-2 sous form d'huile.

Rendement 50%.

Les caractéristiques spectrales du tert-butylamino-1 (hydroxy-4 thiochromannyl-8 oxy)-3 propanol-2 sous forme de base sont les suivantes:

A. Spectre infra rouge, obtenu avec le produit dispersé dans le bromure de potassium:
$V_S$ OH et NH entre 3000 $cm^{-1}$ et 3700 $cm^{-1}$;
$V_S$ C=C 1570 $cm^{-1}$;
$V_S$ C–O–C 1260 $cm^{-1}$ et 1035 $cm^{-1}$.

B. Spectre de résonnance magnétique nucléaire du proton enregistré en 200 $MH_Z$ en solution dans le deuterochloroforme: 1,2 ppm s 9H; 2,0 ppm m 1H; 2,4 ppm m 1H; 3,0 ppm m 3H; 3,25 ppm m 1H; 4,0 ppm m 3H; 5,8 ppm m 1H; 6,75 ppm m 3H; 7 ppm m 3H (3H échangeables par $D_2O$ entre 6,75 et 7 ppm).

C. Spectre de masse enregistré à 80eV, en impact électronique (m/z): 311(M+3,12%), 296(6,06%), 267(10,82%), 182(10,39%), 164(5,11%), 163(6,41%), 114(6,75%), 86 (100%).

L'acétate du tert-butylamino-1 (hydroxy-4 thiochromannyl-8 oxy)-3 propanol-2 est obtenu après addition de 12 ml d'acide acétique glacial à une solution de l'huile obtenue précédemment dans 1 litre d'éther éthylique. Après filtration du précipité formé, lavage à chaud à l'acétonitrile et recristallisation dans un mélange d'acétonitrile et de méthanol (10/1), on obtient 41 g d'acétate. Point de fusion 130–135°C.

Analyse élémentaire de l'acétate($C_{18}H_{29}NO_5S$)
Théorie: C58,19%; H 7,86; N 3,77; S8,63
Trouvé: C58,18%; H 7,58; N 3,80; S8,76.

ETUDE PHARMACOLOGIQUE

Exemple 4

Evaluation de l'activité bêta$_1$-adrénolytique in vitro

Les propriétés bêta-bloquantes du composé de formule I et plus précisément l'intensité de l'activité bêta$_1$-adrénolytique in vitro a été évaluée sur l'inhibition des réponses chronotropes de l'oreillette droite de rat stimulée à l'isoprénaline. Les effets de ce composé ont été comparés à ceux du tertatolol et du propranolol, ce dernier étant un bêta-bloquant de référence.

Les études sont réalisées sur l'oreillette droite prélevée sur des rats Wistar mâles de 300 à 400 g. Après sacrifice, le cœur est prélevé et l'oreillette droite est rapidement disséquée puis placée dans une cuve à organe isolé, contenant une solution physiologique thermostatée à 37°C et oxygénée par un mélange de 95% d'oxygène et 5% de dioxyde de carbone. L'oreillette est reliée à un capteur mécano-électrique. Une tension initiale de 400 mg est appliquée. La fréquence cardiaque de l'oreillette est recueillie à travers un amplificateur-intégrateur. Après une période de stabilisation de 40 mn, dans un premier temps, afin d'établir une courbe contrôle de l'agoniste seul, des doses cumulatives d'isoprénaline ($3.10^{-10}$M, $10^{-9}$M, $3.10^{-9}$ M...) sont ajoutées au bain toutes les 3 mn, jusqu'à l'obtention de l'effet chronotrope maximal. Dans un second temps, afin d'établir la courbe d'intéraction agoniste/antagoniste, une concentration du composé de formule I, de tertatolol ou de propranolol est ajoutée au bain, 10 mn avant l'adjonction des doses cumulatives d'isoprénaline ($10^{-9}$M, $3.10^{-8}$M...) Une seule concentration d'antagoniste est testée par préparation.

La figure 1 représente les courbes dose-réponse de l'isoprénaline en présence et en absence du propranolol.
[ A ] = la concentration molaire d'ISOPRENALINE (échelle logarythmique)
Effet = le pourcentage d'effet par rapport à l'effet maximum sur rythme (b.p.m.)
$C_{AN}$ = concentrations d'antagoniste

La figure 2 représente les courbes dose-réponse de l'isoprénaline en présence et en absence du tertatolol.
[ A ] = la concentration molaire d'ISOPRENALINE (échelle logarythmique)
Effet = le pourcentage d'effet par rapport à l'effet maximum sur rythme (b.p.m.).
$C_{AN}$ = concentrations d'antagoniste

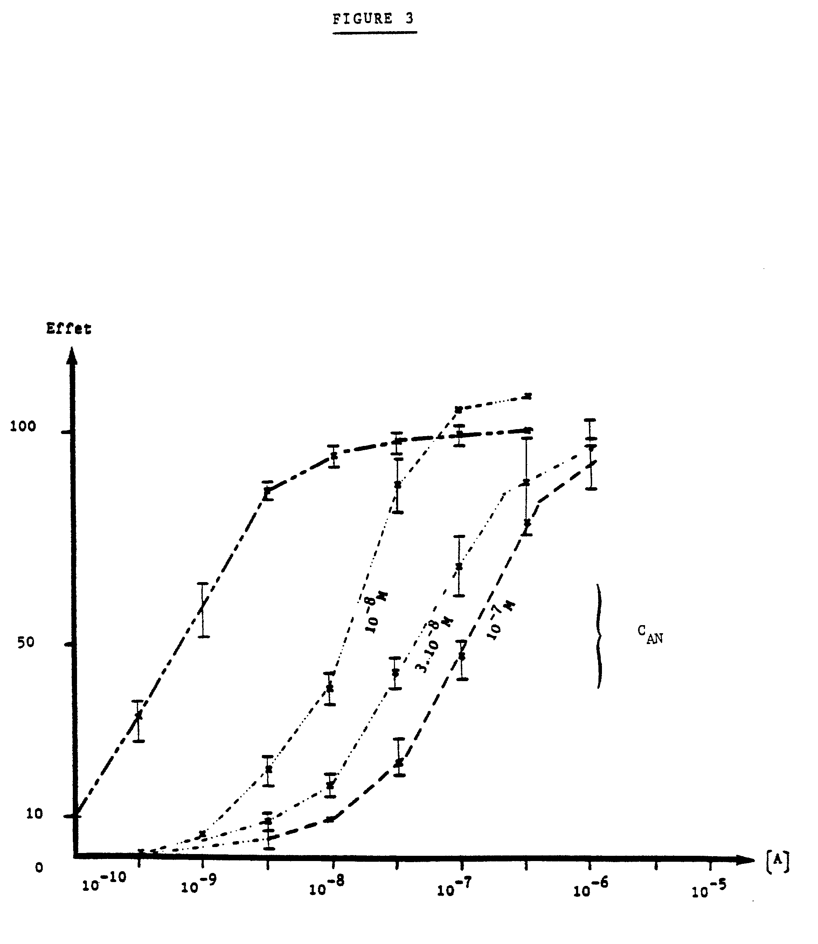

La figure 3 représente les courbes dose-réponse de l'isoprénaline en présence et en absence du composé de formule I.
[ A ] = la concentration molaire d'ISOPRENALINE (échelle logarythmique)
Effet = le pourcentage d'effet par rapport à l'effet maximum sur rythme (b.p.m.).
$C_{AN}$ = concentrations d'antagoniste

Le calcul des $pA_2$, permettant d'évaluer quantitativement un antagonisme compétitif (Guidicelli J.F. J.Pharmacol.(Paris) 1971, 2(3), 373) est appliqué aux trois produits. Le $pA_2$ représente le cologarithme de la concentration molaire de l'antagoniste qui nécessite le doublement de la concentration d'agoniste, pour qu'une réponse de même intensité que celle enregistrée avec l'agoniste utilisé seul soit retrouvée.
Pour chaque expérience, le $pA_2$ est évalué selon la méthode de Van Rossum (Arch. Int. Pharmacodyn (1963)143, 299) et en applicant l'équation $pA_2 = pAx + \log(x-1)$.

pAx: représente le cologarithme de la concentration molaire de l'antagoniste.

x: représente le déplacement en mm de la courbe. (valeur de log (x–1) donné par la table de Van Rossum).

Des tests de Student (series appariées) sont réalisés pour autoriser le moyennage des courbes contrôles et des courbes tests.

Les tableaux 1, 2 et 3 résument les valeurs de $pA_2$ évaluées pour le propranolol, le tertatolol et le composé de formule I respectivement, lors des différents essais.

Tableau 1

| Concentration antagoniste (M) | pAx | x | log (x–1) | pA2 |
|---|---|---|---|---|
| $3.10^{-8}$ | 7,52 | 30,5 | 0,97 | 8,49 |
| $3.10^{-8}$ | 7,52 | 28,5 | 0,9 | 8,42 |
| $3.10^{-8}$ | 7,52 | 28,5 | 0,9 | 8,42 |
| $10^{-7}$ | 7 | 36 | 1,7 | 8,17 |
| $10^{-7}$ | 7 | 26,5 | 0,82 | 7,82 |
| $10^{-7}$ | 7 | 25,5 | 0,78 | 7,78 |
| $10^{-7}$ | 7 | 33,5 | 1,98 | 8,08 |
| $3.10^{-7}$ | 6,52 | 56,0 | 1,86 | 8,38 |
| $3.10^{-7}$ | 6,52 | 68,0 | 2,26 | 8,78 |
| $3.10^{-7}$ | 6,52 | 55,0 | 1,83 | 8,35 |
| $3.10^{-7}$ | 6,52 | 57,0 | 1,89 | 8,41 |
| Propranolol $p\overline{A}_2 = 8,28 \pm 0,089$ (n = 11) | | | | |

Tableau 2

| Concentration antagoniste (M) | pAx | x | log (x–1) | pA2 |
|---|---|---|---|---|
| $10^{-9}$ | 9 | 6 | –0,23 | 7,77 |
| $10^{-9}$ | 9 | 7 | –0,15 | 8,85 |
| $10^{-9}$ | 9 | 3,5 | –0,51 | 8,49 |
| $3.10^{-9}$ | 8,52 | 25,5 | 0,78 | 9,3 |
| $3.10^{-9}$ | 8,52 | 27 | 0,84 | 9,36 |
| $3.10^{-9}$ | 8,52 | 19,5 | 0,54 | 9,06 |
| $10^{-8}$ | 8 | 41 | 1,35 | 9,35 |
| $10^{-8}$ | 8 | 46 | 1,52 | 9,52 |
| $10^{-8}$ | 8 | 47 | 1,55 | 9,55 |
| $3.10^{-8}$ | 7,52 | 35 | 1,13 | 8,65 |
| $3.10^{-8}$ | 7,52 | 32,5 | 1,05 | 8,57 |
| Tertatolol $p\overline{A}_2 = 9,04 \pm 0,118$ (n = 11) | | | | |

Tableau 3

| Concentration antagoniste (M) | pAx | x | log.(x–1) | pA2 |
|---|---|---|---|---|
| $10^{-8}$ | 8 | 37,5 | 1,22 | 9,22 |
| $10^{-8}$ | 8 | 38 | 1,24 | 9,24 |
| $10^{-8}$ | 8 | 31 | 0,99 | 8,99 |
| $3.10^{-8}$ | 7,52 | 55,5 | 1,84 | 9,36 |
| $3.10^{-8}$ | 7,52 | 57 | 1,89 | 9,41 |
| $3.10^{-8}$ | 7,52 | 50 | 1,66 | 9,18 |
| $3.10^{-8}$ | 7,52 | 73 | 2,43 | 9,95 |
| $10^{-7}$ | 7 | 56 | 1,86 | 8,86 |
| $10^{-7}$ | 7 | 63 | 2,10 | 9,10 |
| $10^{-7}$ | 7 | 69,5 | 2,31 | 9,31 |
| $10^{-7}$ | 7 | 61 | 2,03 | 9,03 |
| Composé de formule I $p\overline{A}_2 = 9,24 \pm 0,087$ (n = 11) | | | | |

Le modèle expérimental utilisé a permis l'évaluation des $pA_2$ du propranolol du tertatolol et du composé de formule I. Cette mesure, in vitro, de la "puissance" des antagonistes indique leur propre potentialité.

Le propranolol a servi de molécule de référence; le $p\bar{A}_2$ obtenu est de 8,28 ± 0,089, valeur proche de celle donnée dans la littérature. Les courbes obtenues avec le tertatolol et le composé de formule I, (simple déplacement vers la droite) permettent d'affirmer que l'antagonisme isoprénaline-tertatolol ou isoprénaline composé de formule I est de type compétitif et donc qu'un seul type de récepteur est mis en jeu dans ce mécanisme.

Pour le tertatolol, le $p\bar{A}_2$ correspondant est de 9,04 ± 0,118. Ce composé ayant une structure très différente du propranolol, se révèle donc être 6 fois plus puissant que ce dernier bêta-bloquant. Par contre, d'une manière fort surprenante, le composé de formule I, très proche du tertatolol, a une activité bêta$_1$-antagoniste 2 fois supérieure à celle du tertatolol.

Le $p\bar{A}_2$ obtenu est de 9,24 ± 0,087.

PREPARATION PHARMACEUTIQUE

Exemple 5

Gélules dosées à 0,005 g de tert-butylamino-1 (hydroxy-4 thiochromannyl-8 oxy)-3 propanol-2

Tert-butylamino-1 (hydroxy-4 thiochromannyl-8 oxy)-3 propanol-2 0,0050 g
Amidon de maïs 0,0320 g
Cellulose microcristalline 0,0262 g
Lactose 0,0720 g
Silice colloïdale 0,0003 g
Stéarate de magnésium 0,0015 g
Talc 0,0030 g
pour une gélule blanche taille n°3.

**Revendications pour les états contractants: BE, CH, DE, FR, GB, IT, LI, LU, NL, SE**

1. Composé de formule I:

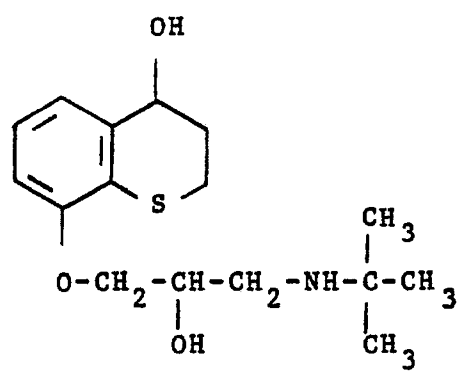

(I)

sous forme racémique ou d'isomères optiques et ses sels d'addition à un acide minéral ou organique pharmaceutiquement acceptable.

2. Procédé de préparation du composé de formule I, caractérisé en ce que l'on réduit le méthoxy-8 thiochromannone-4 de formule II:

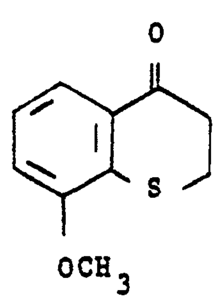

(II)

par un hydrure de bore en méthoxy-8 thiochromannol-4 de formule III:

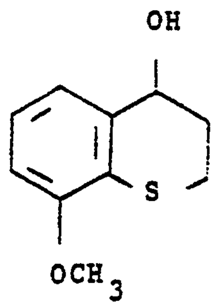

(III)

qu'on déméthyle ensuite, à l'aide d'un thiolate pour obtenir l'hydroxy-8 thiochromannol-4 de formule IV:

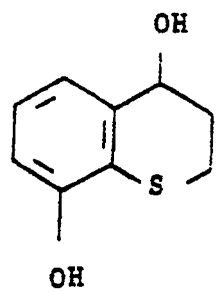

(IV)

sur lequel on fait réagir le chloro-1 époxy-2,3 propane pour former l'(hydroxy-4 thiochromannyl-8 oxy)-3 époxy-1,2 propane de formule V:

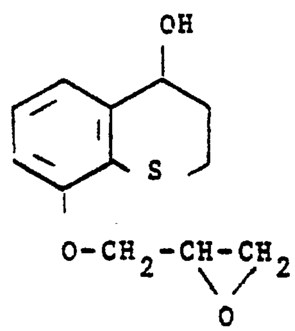

V

et que l'on condense avec la tert-butylamine pour obtenir le composé de formule générale I, et qu'ensuite il est possible de former ses sels d'addition par action d'un acide minéral ou organique pharmaceutiquement acceptable,
ou de le séparer à ses diastéréoisomères qui à leur tour peuvent être salifiés.

3. Compositions pharmaceutiques contenant comme principe actif le composé selon la revendication 1, en association ou en mélange avec un excipient ou un véhicule inerte non toxique, pharmaceutiquement acceptable.

4. Composition pharmaceutique selon la revendication 3 renfermant le principe actif à la dose de 0,1 à 5 mg.

5. Composition pharmaceutique selon les revendications 3 et 4 caractérisée en ce qu'elle renferme aussi un autre principe actif d'action complémentaire ou synergique.

6. Composition pharmaceutique selon les revendications 3 à 5 utilisable dans le traitement des maladies cardiovasculaires et de l'hypertension.

**Revendications pour l'état contractant: AT**

1. Procédé de préparation du composé de formule I:

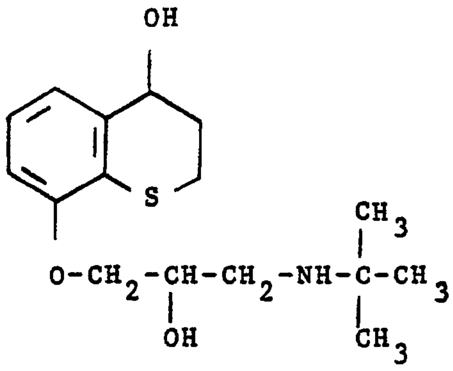

(I)

sous forme racémique ou d'isomères optiques et ses sels d'addition à un acide minéral ou organique pharmaceutiquement acceptable,
caractérisé en ce que l'on réduit le méthoxy-8 thiochromannone-4 de formule II:

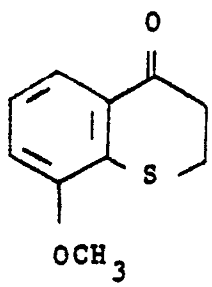

(II)

par un hydrure de bore en méthoxy-8 thiochromannol-4 de formule III:

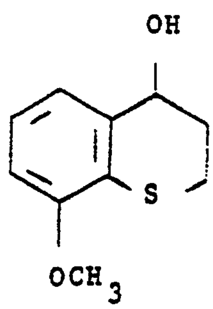

(III)

qu'on déméthyle ensuite, à l'aide d'un thiolate pour obtenir l'hydroxy-8 thiochromannol-4 de formule IV:

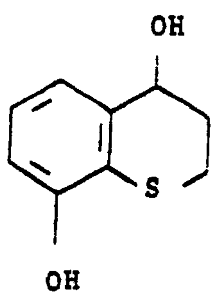

(IV)

sur lequel on fait réagir le chloro-1 époxy-2,3 propane pour former l'(hydroxy-4 thiochromannyl-8 oxy)-3 époxy-1,2 propane de formule V:

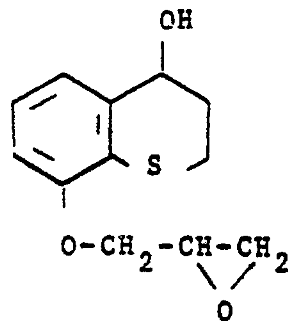

V

et que l'on condense avec la tert-butylamine pour obtenir le composé de formule générale I, et qu'ensuite il est possible de former ses sels d'addition par action d'un acide minéral ou organique pharmaceutiquement acceptable,
ou de le séparer à ses diastéréoisomères qui à leur tour peuvent être salifiés.

**Patentansprüche für die Vertragsstaaten: BE, CH, DE, FR, GB, IT, LI, LU, NL, SE**

1. Verbindung der Formel I:

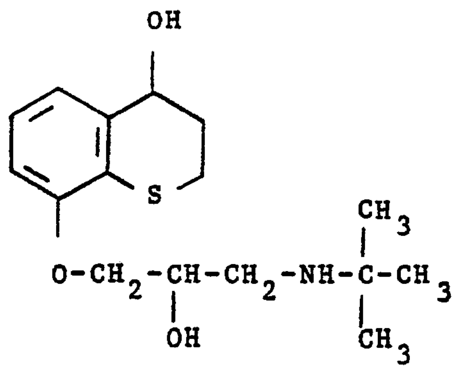

(I)

in Form des Racemats oder der optischen Isomeren und ihre Additionssalze mit einer pharmazeutisch annehmbaren anorganischen oder organischen Säure.

2. Verfahren zur Herstellung der Verbindung der Formel I, dadurch gekennzeichnet, daß man 8-Methoxy-thiochroman-4-on der Formel II:

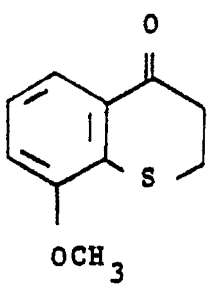

(II)

mit einem Borhydrid zu 8-Methoxy-thiochroman-4-ol der Formel III reduziert:

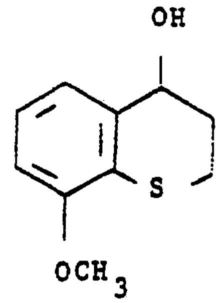

(III)

welches man anschließend mit Hilfe eines Thiolats zu 8-Hydroxy-thiochroman-4-ol der Formel IV demethyliert:

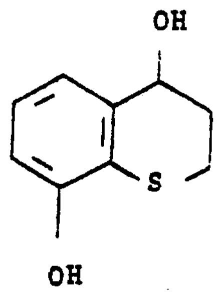

(IV)

welches man mit 1-Chlor-2,3-epoxy-propan zur Bildung von 3-(4-Hydroxy-thiochroman-8-yl-oxy)-1,2-epoxy-propan der Formel V reagieren läßt:

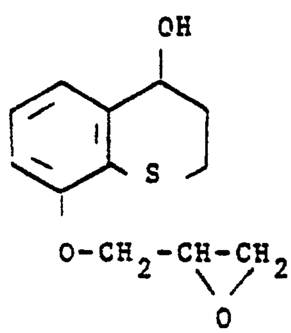

V

und es mit tert.-Butylamin zur Bildung der Verbindung der allgemeinen Formel I kondensiert und anschließend gegebenenfalls ihre Additionssalze durch Einwirkung einer pharmazeutisch annehmbaren anorganischen oder organischen Säure bildet, oder sie in ihre Diastereoisomeren, die der Reihe nach in Salze überführt werden können, auftrennt.

3. Pharmazeutische Zubereitung, enthaltend eine Verbindung nach Anspruch 1 als Wirkstoff, in Kombination oder in Mischung mit einem pharmazeutisch annehmbaren inerten, nicht-toxischen Hilfsstoff oder Träger.

4. Pharmazeutische Zubereitung nach Anspruch 3, enthaltend den Wirkstoff in einer Menge von 0,1 bis 5 mg.

5. Pharmazeutische Zubereitung nach Anspruch 3 und 4, dadurch gekennzeichnet, daß sie zusätzlich einen weiteren Wirkstoff mit ergänzender oder synergistischer Wirkung einschließt.

6. Pharmazeutische Zubereitung nach Anspruch 3 bis 5 zur Verwendung bei der Behandlung von Herzgefäßerkrankungen und Hypertension.

**Patentansprüche für den Vertragsstaat: AT**

1. Verfahren zur Herstellung der Verbindung der Formel I:

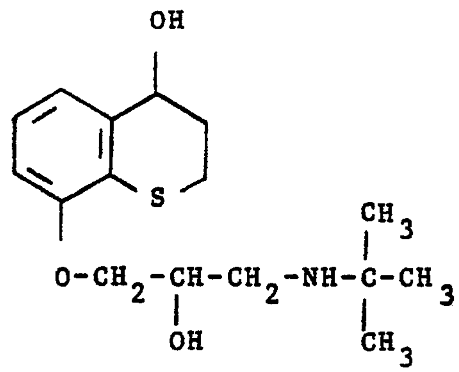

(I)

in Form des Racemats oder der optischen Isomeren und ihre Additionssalze mit einer pharmazeutisch annehmbaren anorganischen oder organischen Säure, dadurch gekennzeichnet, daß man 8-Methoxy-thiochroman-4-on der Formel II:

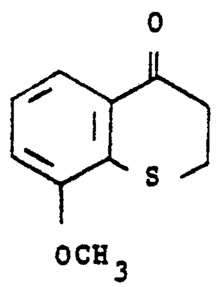

(II)

mit einem Borhydrid zu 8-Methoxy-thiochroman-4-ol der Formel III reduziert:

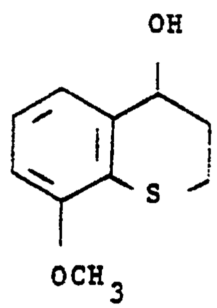

(III)

welches man anschließend mit Hilfe eines Thiolats zu 8-Hydroxy-thiochroman-4-ol der Formel IV demethyliert:

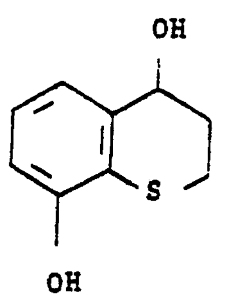

(IV)

welches man mit 1-Chlor-2,3-epoxy-propan zur Bildung von 3-(4-Hydroxy-thiochroman-8-yl-oxy)-1,2-epoxy-propan der Formel V reagieren läßt:

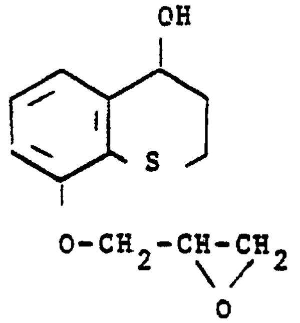

V

und es mit tert.-Butylamin zur Bildung der Verbindung der allgemeinen Formel I kondensiert und anschließend gegebenenfalls ihre Additionssalze durch Einwirken einer pharmazeutisch annehmbaren anorganischen oder organischen Säure bildet, oder sie in ihre Diastereoisomeren, die der Reihe nach in Salze überführt werden können, auftrennt.

**Claims for the Contracting States: BE, CH, DE, FR, GB, IT, LI, LU, NL, SE**

1. Compound of the formula I:

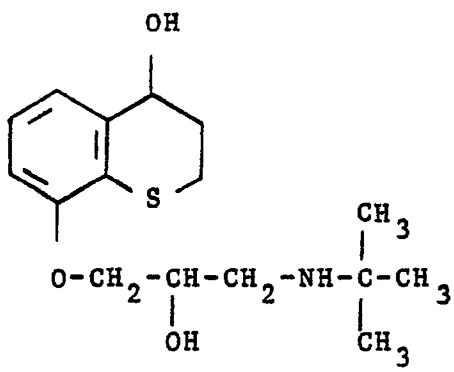

(I)

in racemic form or in the form of optical isomers and addition salts thereof with a pharmaceutically acceptable mineral or organic acid.

2. Process for the preparation of the compound of the formula I, characterised in that 8-methoxy-4-thiochromanone of the formula II:

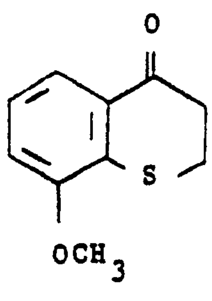

(II)

is reduced by means of a boron hydride to 8-methoxy-4-thiochromanol of the formula III:

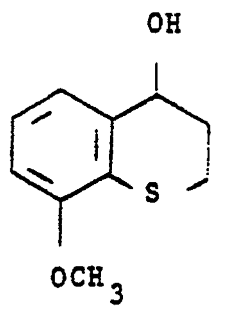

(III)

which is then demethylated, by means of a thiolate, to obtain 8-hydroxy-4-thiochromanol of the formula IV:

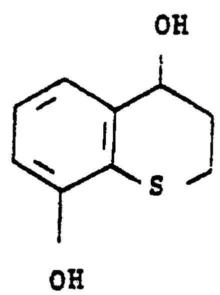

(IV)

with which there is reacted 1-chloro-2,3-epoxypropane to yield 3-(4-hydroxy-8-thiochromanyloxy)-1,2-epoxypropane of the formula V:

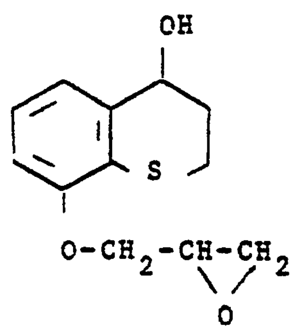

V

and this is condensed with tert.-butylamine to obtain the compound of the general formula I, and it is then possible to form its addition salts by the action of a pharmaceutically acceptable mineral or organic acid, or to separate it into its diastereoisomers which in turn may be converted into salts.

3. Pharmaceutical compositions containing as active ingredient the compound according to claim 1, in association or in admixture with a pharmaceutically acceptable, inert, non-toxic excipient or carrier.

4. Pharmaceutical composition according to claim 3, containing the active ingredient in an amount of from 0.1 to 5 mg.

5. Pharmaceutical composition according to claims 3 and 4, characterised in that it also contains another active ingredient having a complementary or synergic action.

6. Pharmaceutical composition according to claims 3 to 5, which can be used in the treatment of cardiovascular diseases and hypertension.

**Claims for the Contracting State: AT**

1. Process for the preparation of the compound of the formula I:

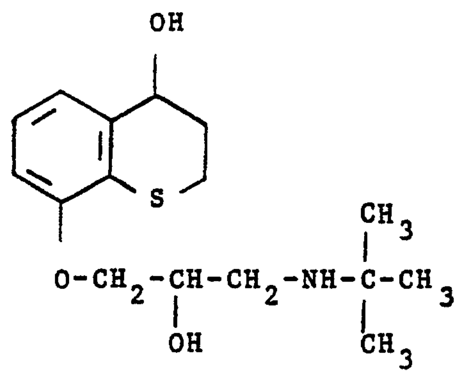

(I)

in racemic form or in the form of optical isomers and addition salts thereof with a pharmaceutically acceptable mineral or organic acid, characterised in that 8-methoxy-4-thiochromanone of the formula II:

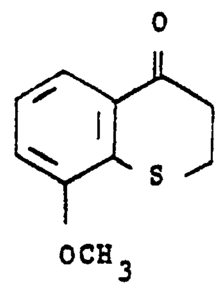

(II)

is reduced by means of a boron hydride to 8-methoxy-4-thiochromanol of the formula III:

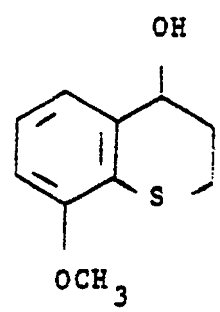

(III)

which is then demethylated, by means of a thiolate, to obtain 8-hydroxy-4-thiochromanol of the formula IV:

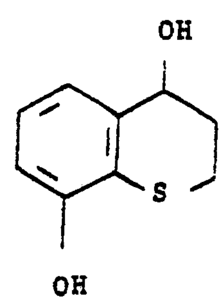

(IV)

with which there is reacted 1-chloro-2,3-epoxypropane to yield 3-(4-hydroxy-8-thiochromanyloxy)-1,2-epoxypropane of the formula V:

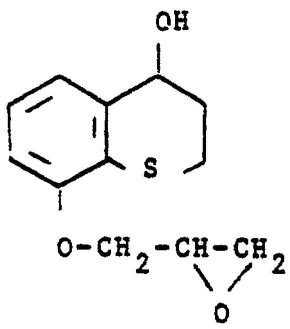

V

and this is condensed with tert.-butylamine to obtain the compound of the general formula I, and it is then possible to form its addition salts by the action of a pharmaceutically acceptable mineral or organic acid, or to separate it into its diastereoisomers which in turn may be converted into salts.

FIGURE 1

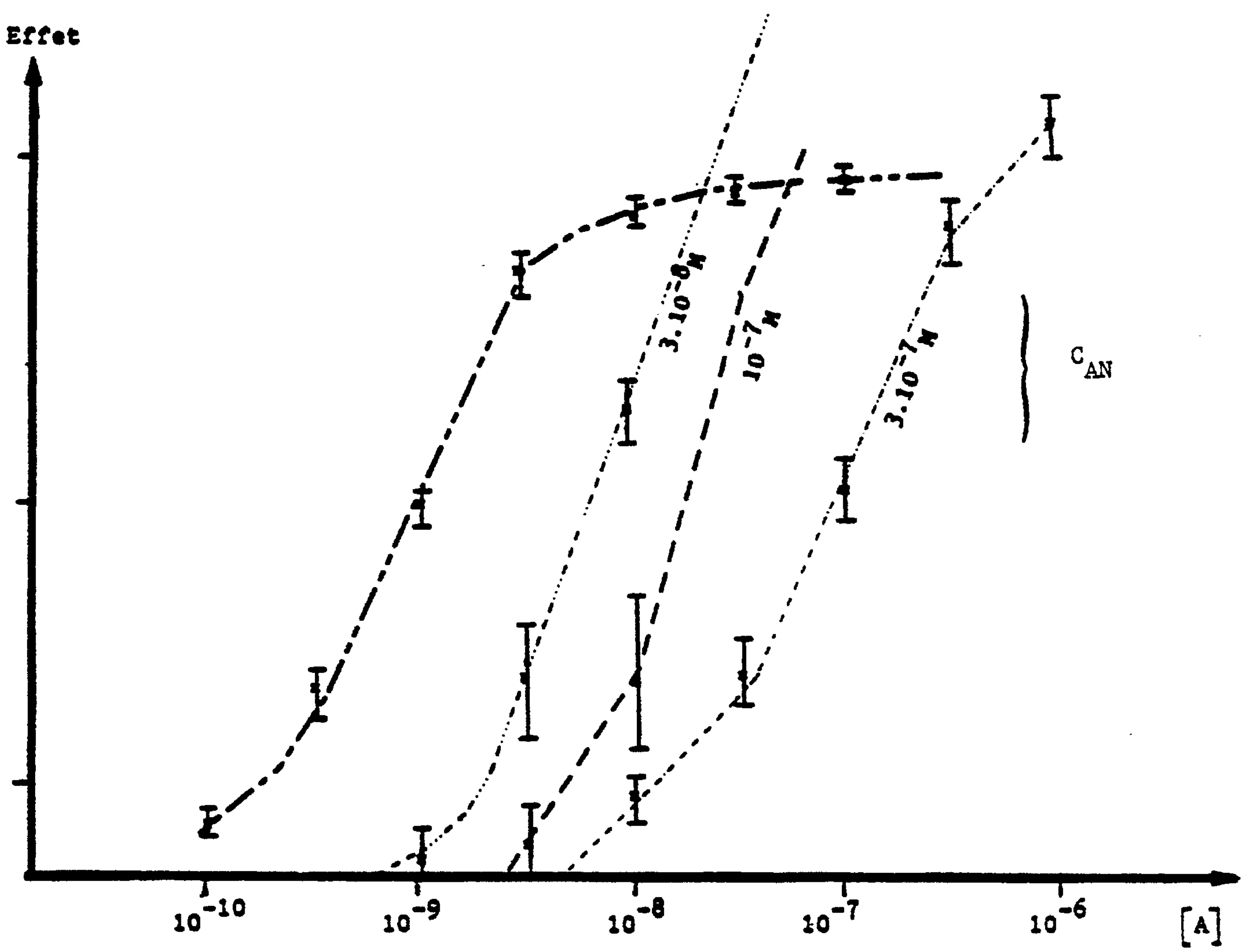

FIGURE 2

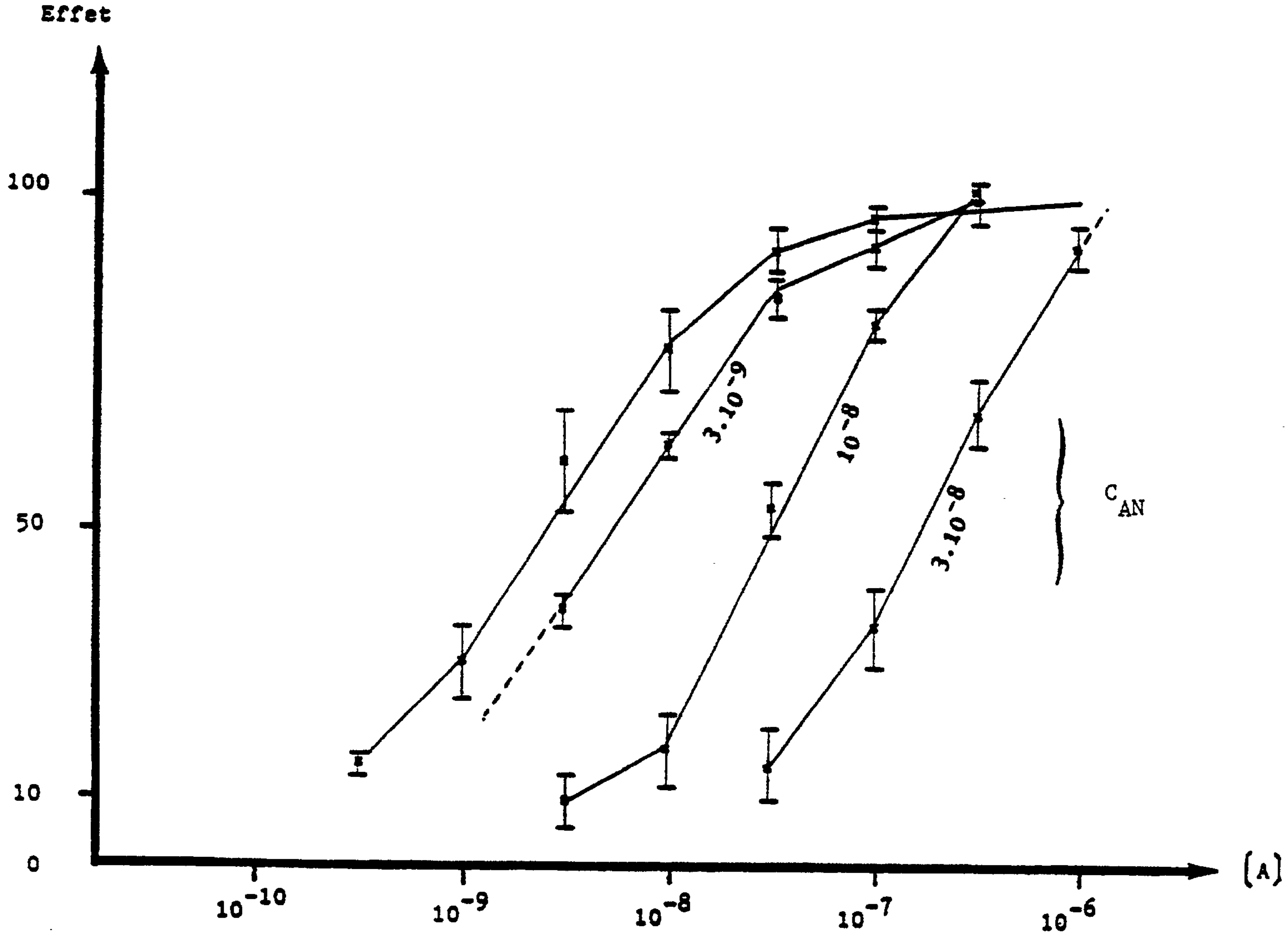

FIGURE 3